# NEUE EUROPÄISCHE PATENTSCHRIFT

(11) **EP 0 496 839 B2**
(45) Veröffentlichungstag und Bekanntmachung des Hinweises auf die Entscheidung über den Einspruch: **03.03.2004**
(45) Hinweis auf die Patenterteilung: 15.05.1996
(21) Anmeldenummer: 91901668.3
(22) Anmeldetag: 17.01.1991
(51) Int. Cl.: A61K 39/385

(54) **IMPFSTOFF GEGEN DROGEN**
VACCINE AGAINST DRUGS OF ABUSE
VACCIN CONTRE LES DROGUES

(30) Priorität: 22.08.1990 CH 272090
(43) Veröffentlichungstag der Anmeldung: 05.08.1992
(73) Patentinhaber: CHILKA Ltd., Road Town, Tortola (VG)
(72) Erfinder: CERNY, Erich Hugo, CH-1204 Genève (CH)
(74) Vertreter: Schweiger, Georg, Dr.
(86) Internationale Anmeldenummer: PCT/CH1991/000016
(87) Internationale Veröffentlichungsnummer: WO 1992/003163

(56) Entgegenhaltungen:
- EP-A- 311 383
- EP-A- 363 041
- DE-A- 2 548 196
- US-A- 4 045 420
- ANNALES PHARMACEUTIQUES FRANCAISES, Band 35, Nr. 7/8, 1977, Paris (FR); PHAM HUY CHUONG et al., Seiten 257-264
- K F Bonese et al (1974) Nature 252, 708-710
- Wainer et al (1972) Science 176, 1143-1145
- Kovalev et al (1980) Khim Pharm, 200-204 (english translation)

## Beschreibung

Die Erfindung betrifft ein Verfahren gemaess Patentanspruch 1 und 2 zur Herstellung eines Impfstoffes gegen Drogen, welche eine Abhängigkeit erzeugen können.

Drogenabhängigkeit wird oft als eine physische und eine psychische Abhängigkeit verstanden. Die eine oder die andere Komponente kann dominieren, aber beide Komponenten finden sich normalerweise bei einem Süchtigen. Drogen welche eine physische Abhängigkeit hervorrufen sind zum Beispiel Opiate, Barbiturate, Alkohol, Zigaretten, angstlösende Medikamente und einige Sedativa. Psychologische Abhängigkeit auf der anderen Seite ist beschrieben als ein Verlangen für eine Droge, das temporär erlischt, wenn der gewünschte Effekt nach Einnahme der Droge auftritt. Beispiele für eine Droge die auch eine psychische Abhängigkeit erzeugen kann sind Opiate und Zigaretten.

Ein Therapieerfolg bedingt eine Befreiung sowohl von der psychischen als auch von der physischen Suchtkomponente. Die klassische Therapie versucht eine Entgiftung durch die sukzessive Verringerung oder in einigen Fällen (Methadon) Substituierung der Droge. Die physische Suchtkomponente wird durch ein Rehabilitierungsprogramm behandelt.

Der Impfstoff der vorliegenden Erfindung erlaubt ein neues Vorgehen zur Therapie und Prävention von Patienten, die von Drogen abhängig sind. Weiterer Gebrauch der Droge in der Gegenwart des Antikörpers nach der Impfung inaktiviert die Droge und stimuliert die Produktion von neuen spezifischen Antikörpern. Der gewünschte Drogeneffekt ist daher eliminiert und der Teufelskreis zwischen Stimulation und Applikation unterbrochen.

Der neue Impfstoff erweitert die Anwendung von Vaccinen auf das Gebiet der Drogen. Die folgenden fundamentalen Unterschiede bestehen zwischen einer klassischen Vaccine und dem vorliegenden Impfstoff: 1) der Impfstoff ist nicht gegen einen infektiösen Erreger sondern gegen eine Droge gerichtet. 2) Die typische Anwendung dieses Impfstoffes ist nicht präventiv sondern therapeutisch 3) Das Antigen gegen das der Impfstoff gerichtet ist, ist in den meisten Fällen selber nicht antigenisch sondern muss an ein Trägerprotein gebunden sein, um Antikörper hervorzurufen. 4) der Abhängigkeit erzeugende Effekt der Droge ist die Interaktion der Droge mit dem Rezeptor. Diese Interaktion ist durch das Binden des Antikörpers mit der Droge unterbunden.

Es ist daher eines der Ziele dieser Erfindung einen Impfstoff gegen Drogen zu beschreiben:
1) der gegen eine oder mehrere Abhängigkeit erzeugende Drogen gerichtet ist und der diese Drogen an eine Trägerprotein gebunden enthält, um die Droge immunogen zu machen.
2) der sowohl gegen Drogen, die eine physische , als auch gegen Drogen, die eine psychische Abhängigkeit erzeugen, wirksam ist.
3) der therapeutisch (der Drogenabhängige ist geimpft um die Droge zu inaktivieren), als auch präventiv ( zum Beispiel um den Fetus einer drogenabhängigen Schwangeren vor der direkten Drogenschädigung als auch späterer Abhängigkeit zu schützen) , verwendet werden kann.
4) der gegen all Arten von Drogen gerichtet sein kann: zum Beispiel Opiate und Opiat- ähnliche Drogen, Marihuana, Kokain, Amphetamine, Antipsychotische Drogen, Barbiturate und andere Sedativa, psychomimetische Drogen, anticholinergische Drogen als auch Substanzen, welche diese Drogen kontaminieren.
5) der typischerweise den B- Zell Arm des Immunsystemes aktiviert, der aber auch eine zusätzliche Wirksamkeit erlangen kann durch eine Aktivierung des T-Zell Armes des Immunsystemes oder durch einen allergenen Effekt .
6) der als ein Komplement zu einer anderen Therapieform verwendet werden kann.

### Definitionen:

Drogen welche eine Abhängigkeit erzeugen koennen: Alle Substanzen, die im weitesten Sinne eine physische oder eine psychische Abhängigkeit nach einer oder mehrmaliger Anwendung hervorrufen. Eine Droge ruft eine physische Abhängigkeit hervor, wenn ein Entzugssymptom nach abruptem Entzug der Droge auftritt. Psychische Abhängigkeit auf der andereen Seite wir hervorgerufen durch eine Droge, an die sich der Abhängige gewöhnt hat und die im Süchtigen ein Verlangen nach einem bestimmten Effekt erzeugt.

Drogen, die Abhängigkeit erzeugen können sind unter anderem: Kokain und Kokainderivate (Kokain, das von Drogenabhängigen konsumiert wird enthält zusätzlich häufig Mannitol, Laktose, Nikotin, Effedrin, Kaffein, Prokain und Amphetamine), Barbiturate und andere Sedativa, Benzodiazepine, Methaqualon, Glutethimid, Chloral Hydrat, Methyprylon, Paraldehyd und Bromide, antipsychotische Drogen, psychomimetische Drogen wie Phenylcyclidin oder LSD (Lysergig Acid Diethylamide), Phenylcyclidin und Analoga, Amphetamin und Tryptamin Derivate, Psylocybin, volatile Nitrite und anticholinergische Drogen.
Komponenten von Drogen : Drogen werden nicht immer in chemisch reiner Form verwendet und sind oft mit anderen Substanzen vermischt. Es'mag unter gewissen Umständen vorteilhaft sein, den Impfstoff gegen mehrere Substanzen gleichzeitig zu richten um einen breiteren Schutzeffekt zu erhalten.
Vaccine: Eine Präparation, bestehend aus einem Immunogen , welche den B-Zell Arm und eventuell auch den T-Zell Arm des Immunsystemes zu aktivieren vermag. Das Immunogen des Impfstoffes dieser Erfindung ist typischerweise eine Droge, welche als Hapten an eine Trägersubstanz gebunden ist. Die Bindung zwischen Hapten und Träger kann kovalent oder ionisch sein oder auf Van der Waals oder Wasserstoff Brücken beruhen. Die Bindung kann aber auch ein oder mehrere Atome als Brücke enthalten. Eine Droge kann auch immunogen gemacht werden durch einfaches chemisches Vernetzen der Droge (zum Beispiel mit Glutaraldehyd) um ein Molekulargewicht über 5000 Dalton zu erhalten. In diesem Fällen kann auf eine Trägersubstanz verzichtet werden.

Der Fabrikationsprozess der Vaccine enthaltet typischerweise zwei Schritte: 1) Konjugation: Die Droge wird an die Trägersubstanz gebunden 2) Purifikation: das Hapten-Trägersubstanz Konjugat wird von Beiprodukten des Kuppelungsprozesses gereinigt und in eine physiologische Lösung gegeben. Die Konjugation kann in organische Lösungsmittel oder in wässerigem Milieu erfolgen. Häufig verwendete Kuppelungssubstanzen sind: Karbodiimide, Imidoester, N-Hydroxysuccinimid Ester oder ihre wasserlöslichen Sulfo- Derivate, Maleimid- Derivate und Phenyl Azide.
Die Purifikation erfolgt üblicherweise durch eine Dialyse oder mit Hilfe von Gel oder lonen Chromatographie. Das Hapten-Trägersubstanz Konjugat hat meistens ein Molekulargewicht über 100 000 Dalton und kann daher leicht von den Verunreinigungen wie zum Beispiel Uberschuss der Kuppelungssubstanz gereinigt werden, die typischerweise ein Molekulargewicht unter 500 Dalton haben. Bevorzugte Trennmethoden sind extensive Dialyse in einem Dialyseschlauch mit einer wässerigen Lösung, beispielsweise mehrmaliger Puffer Wechsel mit Phosphate gepufferter Saline (PBS) oder Chromatographie über eine Sephadex G 25 Kolonne (Pharmacia LKB Biotechnology, Bromma, Schweden). Sterilfiltration mit einem Filter von 0,2 micrometer und Entfernung von pyrogenem Material sind die Endschritte der Purifikation.

### Vaccine für präventive Zwecke:

Die Person, die geimpft werden soll ist noch nicht drogenabhängig. Eine Person geeignet für eine präventive Impfung wäre zum Beispiel ein Säugling, der die Droge über die Muttermilch einnimmt.

### Vaccine für therapeutische Zwecke:

Die Person, die geimpft werden soll ist bereits drogenabhängig oder hat begonnen die Droge zu verwenden. Eine Person geeignet für die Impfung ist beispielsweise ein Morphin Süchtiger, der eine Entziehungskur untergehen will.
Immunisierung gegen Drogen: Bei Patienten, die an Vergiftungs-erscheinungen einer Droge leiden, bleibt keine Zeit um Antikörper durch eine Vaccine zu erzeugen. Solchen Fällen kann durch direkte Applikation von spezifischen humanen oder tierischen Antikörpern geholfen werden. Der spezifische Antikörper bindet sich nach Applikation in kurzer Zeit an die noch ungebundene Droge und die Immunkomplexe werden durch das retikulo-endotheliale System eliminiert. Dadurch wird der Körper entgiftet. Es besteht die Möglichkeit eine Mischung von Antikörpern gegen mehrere Drogen zu verwenden, da in vielen Fällen nicht klar ist, mit welcher Droge sich der Patient vergiftet hat.
Antikörper: Eine Klasse von Plasmaproteinen, die durch die B-Zellen des Immunsystemes nach Stimulation durch ein Antigen erzeugt werden. Humane Antikörper sind Immunoglobuline der Ig G, M A, E oder D Gruppe. Antigene: Eine Substanz, die fähig ist, eine Immunantwort hervorzurufen. Die Antigene sind typischerweise Proteine, aber sie können auch Zucker und Lipidgruppen enthalten. Antigene habe typischerweise ein Molekulargewicht über 10 000 Dalton.
Haptene: Dies sind kleine Moleküle , die für sich selbst nicht Antikörper hervorrufen können. Um antigenisch zu werden muss ein Hapten an einen Träger gebunden werden. Die Immunantwort gegen ein Hapten kann gegen das Hapten gerichtet sein, gegen den Träger oder gegen beide Substanzen. Das Hapten der vorliegenden Erfindung ist normalerweise eine Droge, ein Metabolit der Droge oder ein Bestandteil einer Droge wie zum Beispiel eine Komponente von Zigarettenrauch.
Einige Drogen sind schnell metabolisiert nach Aufnahme durch den Körper. Morphin zum Beispiel ist schnell in Morphin-3-Glucuronat und Morphin-6-Glucuronat umgewandelt. Es mag daher in gewissen Fällen von Vorteil sein, wenn Metaboliten als Hapten verwendet werden an Stelle der ursprünglichen Droge.
Trägersubstanz: Das Problem Antikörper gegen ein kleines Molekül (Hapten) hervorzurufen wird dadurch gelöst, dass das kleine Molekül an einen Träger gebunden wird. Diese Bindung macht das Hapten immunogen, das heisst es werden nach Injektion in den Körper Antikörper hervorgerufen. Die Bindung des Haptens an das Trägerprotein ist oft kovalent, kann aber auch ionisch sein oder über eine chemische Komponente erfolgen, die als Brücke zwischen Hapten und Trägersubstanz dient. Die Trägersubstanz ist typischerweise ein Protein, kann aber auch Zucker und Lipide in mono- oder polymerer Form enthalten. Unter besonderen Umständen ist es auch möglich das Hapten zu vernetzen so dass das Hapten keine Trägersubstanz mehr benötigt um immunogen zu werden.
Antigen/ Antikörper Interaktion: Die Kombination von einem spezifischen Antikörper mit der Oberflächen Determinante von einem korrespondierenden Antigen ( = Hapten plus Trägersubstanz) ist reversibel und der Komplex kann dissoziieren in Abhängigkeit von der Bindungsstärke Dadurch dass die beiden Partner eine komplementäre Form der Elektronenwolken an der Oberfläche haben (ähnlich wie ein Schlüssel mit dem Schloss) gibt es eine Interaktion, welche durch die Wasserstoffbrücken, die Van der Waals Kraft, die electrostatische Kraft und die hydrophobe Interaktion determiniert ist.
Adjuvans: Dies ist eine Substanz oder Mischung von Substanzen, die zur Vaccine dazugegeben werden um die Effizienz der Antikörperbildung zu erhöhen oder um sicherzustellen, dass eine bestimmte Klasse von Antikörpern wie IgM Immunoglobuline oder Komplement bindende Antikörper erzeugt werden. Substanzen welche als Adjuvantien gebraucht werden, sind beispielsweise Mineralöle, Derivate von Aluminium oder Bestandteile von Mykobakterien. Die Vaccinen dieser Erfindung können mit oder ohne Adjuvantien verwendet werden.
Applikationsarten: Die Vaccine kann intravenös, intramusculär, subkutan , oder intradermal iniziert werden. Sie kann aber auch oral (z. B. Kapsel, die gegen Verdauung im Magen und Dünndarm schützt) verabreicht werden. Die Vaccine kann ebenfalls in bestimmten Fällen als Aerosol gegeben werden oder auf der Haut zur perkutanen Resorption verabreicht werden. Die Vaccine kann einmal oder mehrere Male verabreicht werden.

Um das Verständniss der Erfindung noch zu verbessern, sind folgende Beispiele angegeben.

### Beispiel 1:

Dieses Beispiel zeigt am Mausmodell wie nach Impfung Antikörper gegen Morphinderivate oder Barbiturate erzeugt werden und die Droge nach Injektion innert kurzer Zeit aus dem Kreislauf entfernt wird.

### Hapten-Trägersubstanz Konjugation:

Die Methode von Wainer wird verwendet um das Morphin-6-Hemisuccinat zu synthetisieren und an Bovines Serum Albumin (BSA) zu binden (Wainer B.H. et al. , 1972, Science 176, 1143-45 , Wainer B. H. et al., 1972, Science 178, 647-8). Eine andere Präparation wird zubereitet indem Morphin zu 3-o-Karboxymethylmorphin umgewandelt wird durch Reaktion der freien Base mit Sodium-beta-Chlorazetat in absolutem Alkohol. Karboxymethylmorphin (8 mg) wird dann in 2 ml von destilliertem Wasser welches 10 mg BSA enthält aufgelöst und 8 mg von 1-Athyl-3-(3-dimethylaminopropyl)Karbodiimid werden dazugegeben nach Ajustieren des pH der Lösung auf 6. Die Mischung wird nach Inkubation in Raumtemperatur über Nacht extensiv gegen PBS , pH 7,6 dialysiert.

Die folgende Methode wir verwendet um Barbiturat - Keyhol Lympet Hemocyanin (KLH, Sigma, St. Louis, USA) Konjugate für die Vaccine zu erhalten: 5-allyl-5-(1-karboxyisopropyl)barbitursäure wird umgewandelt zu 5-allyl-5-(1-p-nitrophenyloxykarbonylisopropyl)barbitursäure durch Reaktion von 10 mg von der freien Base mit p-Nitrophenol (12 mg) in einer N,-N-Dimethylformamid Lösung während 24 Stunden bei 4° C. Die Kupplung an KLH (19 mg) wird dann in einer Lösung ausgeführt die gleiche Volumina von Glycerin und Wasser und 10 mg Dicyclohehylkarbodiimid enthält. Die Mischung ist nach 24 stündiger Inkubation bei 4° C mit mehrmaligem Pufferwechsel gegen PBS pH 7.6 dialyziert. Der Substitutionsgrad der Droge an der Trägersubstanz wird berechnet als ein Ansteigen der Absorption vom Konjugat bei 202 nm verglichen mit einer KLH Kontrollösung (molarer Extinktionskoeffizient der Barbiturate = 19500).

Immunisation: 5 weibliche Balb/ C Mäuse werden subkutan 8 mal in 2 wöchigen Intervallen mit 40 mikrogramm des Morphin-BSA Konjugates per Maus und Dose iniziert. Eine weitere Gruppe von 5 weiblichen Balb/C Mäusen erhält das Barbitur at-KLH Konjugat unter den gleichen Bedingungen, und eine Kontrollgruppe von 10 gleichaltrigen weiblichen Balb/C Mäusen wird unter den gleichen Konditionen mit dem Puffer des Impfstoffes ohne Vaccine behandelt.

### Blutproben: Die Blutproben werden von der Schwanzvene entnommen.

Messung des Drogengehaltes: Die High Pressure Liquid Chromato-graphy (HPLC) Methode von Joel et al. (1988, Chromatography, 430: 394-9 wird als Referenzmethode verwendet um Morphin, Morphin-6-Glucuronid und Morphin-3-Glucuronid zu messen. Die Morphine, als auch die Barbiturate werden auch mit einem kommerziellen Enzyme Multiplikation Immunoassay (EMIT, Syva Corp., Palo Alto, USA) in Uebereinstimmung mit der Gebrauchsanweisung (EMIT operator's manual, Shiva Corporation, Palo Alto, USA) bestimmt. Es ist für die Evaluierung der Vaccine wichtig, freie Droge von Antikörper gebundener Droge zu differenzieren: der EMIT Test wird daher folgendermassen modifiziert: die verdünnte Serumprobe wird vor Durchführung des EMIT Testes von Antikörpern gereinigt, durch eine zwei stündige Inkubation bei Zimmer Temperatur in einem PBS Puffer, pH 7,6 , der CnBr- aktivierte Sepharose 4 B enthält, an welche polyklonale Kaninchenantikörper gegen humane leichte Ketten der Immunoglobuline gebunden sind (Pharmacia LKB Biotechnology, Bromma, Schweden).

Fünf Mäuse, die geimpft wurden und fünf Mäuse der Kontrollgruppe werden 2 Wochen nach der letzten Immunisation mit 0,5 mg Morphin pro Maus iniziert. Die Serumproben werden unmittelbar nach der Injektion und 2 Stunden nach der Injektion entnommen und die Menge der Droge im Serum wird mit Hilfe des modifizierten EMIT Testes bestimmt. Die Droge kann in den Proben, die gerade nach Injektion der Droge entnommen wurden noch festgestellt werden, ist aber 2 Stunden nach Injektion nicht mehr feststellbar. Die Diskrimination im Testsystem zwischen positiver und negativer Probe wir sowohl für die Barbiturate als auch Morphin auf 5 ng/ml festgelegt. Die Interpretation der Testresultate ist, dass nach erfolgreicher Impfung, sich die spezifischen Antikörper an die Droge gebunden haben und die freie Droge daher nicht mehr feststellbar ist.

### Beispiel 2:

Dieses Experiment demonstriert, dass die "Immunität" gegen die Droge auch nach Monaten noch vorhanden ist. Zwei der immunisierten Mäuse von Beispiel 1 und drei Mäuse der Kontrollgruppe werden 2 und 4 Monate nach dem ersten Experiment mit 0,5 mg Morphin iniziert. Eine Blutprobe wird wie im ersten Experiment unmittelbar nach der Injektion und 2 Stunden nach der Injektion entnommen und sad Serum wird im EMIT Test für Morphin getestet.

Das Serum der Mäuse, welche vorher mit dem Morphin-BSA Konjugat getestet wurden, ist wieder negativ und nur die Proben, die unmittelbar nach der Injektion entnommen werden, zeigen ein positives Resultat. Die Kontrollgruppe zeigt in allen Untersuchungen erkennbare Spuren von Morphin. Es wird der Schluss gezogen, dass die Vaccine über längere Zeit wirksam ist.

### Beispiel 3:

Dieses Beispiel dient dazu, die Schutzwirkung der spezifischen Antikörper, respektive des spezifischen Immunserums welches die Antikörper enthält, zu zeigen.

Jeweils zwei Mäuse der Kontrollgruppe erhalten 0,5 mg Morphin (Maus A und B) und 0.5 mg Phenobarbital (Maus C und D). Maus A und C erhalten jeweils 0,5 ml des Serums der Mäuse welche vorher mit dem Anti-Morphin Impfstoff geimpft wurden und Maus B und D erhalten o,5 ml des Serums der Mäuse welche vorher mit dem Anti-Barbiturat Impfstoff geimpft wurden. Zwei Stunden nach der Injektion des Immunserums werden Blutproben entnommen und für die Gegenwart der Drogen untersucht (modifizierter EMIT Test). Die Maus welche Morphin und anti-Morphin Serum bekommen hat, zeigt keine freie Droge im Test. Die Maus welche Morphin und anti-Barbiturat Serum bekommen hat, zeigt freies Morphin im Serum. Das analoge Phänomen zeigt sich bei den Mäusen welche Phenobaribal erhalten haben: bei der Maus welche das spezifische Immunserum erhalten hat lässt sich keine freie Droge mehr nachweisen (Maus D). Die Maus mit dem Anti-Morphin Serum (Maus C) zeigt immer noch Spuren des Barbiturates im Serum.

Dieses Experiment kann auch mit Antiserum durchgeführt werden, dass aus monoklonalen Antikörpern oder einer Mischung von monoklonalen Antikörpern gegen die Drogen besteht: es wird ein analoges Resultat erwartet.

## Patentansprüche

1. Verfahren zur Herstellung eines therapeutisch und/oder präventiv verwendbaren Impfstoffs gegen wenigstens eine Abhängigkeit erzeugende Droge,
wobei man
a) eine oder mehrere Drogen als Hapten mit einer Trägersubstanz konjugiert, um das Hapten antigenisch zu machen;
b) das Produkt aus Stufe a) gegebenenfalls reinigt; und
c) zu einem Impfstoff formuliert, welcher nach Impfung eines Patienten bewirkt, dass bei weiterem Gebrauch der Droge in Gegenwart von Drogenspezifischen Antikörpern die Droge inaktiviert wird und neue Drogenspezifische Antikörper produziert werden; wobei der Impfstoff den Drogeneffekt im Patienten eliminiert und damit den Kausalzusammenhang zwischen Drogenstimulation und Drogenapplikation unterbricht; und wobei der durch die Impfung erzielte, die Drogenwirkung eliminierende Effekt einem Effekt entspricht, der durch achtmalige subkutane Gabe von 40µg eines Morphin-BSA- oder Barbiturat-KLH-Konjugates in zweiwöchigen Intervallen in Balb/C-Mäusen induzierbar ist.

2. Verfahren nach Anspruch 1, **dadurch gekennzeichnet, dass** die Droge, welche eine Abhängigkeit erzeugt, zu einer der folgenden Gruppen gehört: Opiate, Marihuana, Amphetamine, Kokain, Barbiturate, Sedativa, Methaqualon, Benzodiazepine, Lysergsäure Diethylamid, psychomimetische Drogen, Nikotin, anticholinergische Drogen und antipsychotische Drogen.

3. Verfahren nach Anspruch 1, **dadurch gekennzeichnet, dass** die Trägersubstanz eine oder mehre der folgenden chemischen Bestandteile enthält: Proteine, Glycoproteine, Polysacharide, Liposacharide, Lipoproteine, Metalle.

4. Verfahren nach Anspruch 1, **dadurch gekennzeichnet, dass** die Bindung zwischen der Droge, welche eine Abhängigkeit erzeugt und dem Hapten durch eine Brückensubstanz erfolgt.

5. Verfahren nach Anspruch 1, **dadurch gekennzeichnet, dass** man den Impfstoff zusammen mit einem Adjuvans formuliert, um den immunogenen Effekt des Impfstoffs zu verstärken.

6. Verfahren nach Anspruch 1, **dadurch gekennzeichnet, dass** man den Impfstoff durch Sterilfiltration in eine physiologisch verträgliche Form bringt.

7. Verfahren nach Anspruch 1, **dadurch gekennzeichnet, dass** man eine wässrige Lösung verwendet, deren pH-Wert mit einem Puffer zwischen 5,5 und 8 eingestellt worden ist.

8. Verfahren nach Anspruch 1, **dadurch gekennzeichnet, dass** der Impfstoff nach Bindung des Haptens an die Trägersubstanz dialysiert wird, um Beiprodukte der Herstellung zu entfernen.

## Claims

1. Method of producing a vaccine for therapeutic and/or preventative use against at least one dependency-creating drug, wherein
a) one or more drugs is conjugated as a hapten to a carrier substance in order to render the hapten antigenic;
b) the product from step a) is optionally purified; and
c) is formulated as a vaccine, which after vaccination of a patient causes, if the drug is further used, said drug to be inactivated in the presence of drug-specific antibodies and new drug-specific antibodies to be produced; whereby the vaccine eliminates the effect of the drug in the patient and thereby breaks the causal connection between the stimulation by the drug and its administration; and whereby the effect of eliminating the action of the drug which is achieved by means of the vaccination corresponds to an effect inducible by subcutaneous administration of 40 µg of a morphine/BSA or barbiturate/KLH conjugate eight times at two-weekly intervals in Balb/C mice.

2. Method according to claim 1, **characterised in that** the drug which creates a dependency belongs to one of the following groups: opiates, marijuana, amphetamines, cocaine, barbiturates, sedatives, methaqualone, benzodiazepines, lysergic acid diethylamide, psychomimetic drugs, nicotine, anticholinergic drugs and antipsychotic drugs.

3. Method according to claim 1, **characterised in that** the carrier substance contains one or more of the following chemical components: proteins, glycoproteins, polysaccharides, liposaccharides, lipoproteins, metals.

4. Method according to claim 1, **characterised in that** the binding between the drug which creates a dependency and the hapten is via a bridging substance.

5. Method according to claim 1, **characterised in that** the vaccine is formulated together with an adjuvant in order to intensify the immunogenic effect of the vaccine.

6. Method according to claim 1, **characterised in that** the vaccine is brought into a physiologically acceptable form by sterile filtration.

7. Method according to claim 1, **characterised in that** an aqueous solution is used, the pH of which has been adjusted to between 5.5 and 8 using a buffer.

8. Method according to claim 1, **characterised in that** after binding of the hapten to the carrier substance the vaccine is dialysed in order to eliminate by-products of the manufacturing process.

## Revendications

1. Procédé de préparation de vaccins utilisables en thérapie ou en prévention contre au moins une drogue engendrant une dépendance, suivant lequel :
a) on conjugue une ou plusieurs drogues en tant que haptène avec une substance porteuse pour rendre l'haptène antigénique,
b) on purifie le cas échéant le produit de l'étape a),
c) et on en fait une formulation de vaccin qui, après vaccination d'un patient, a pour effet, lors d'une poursuite de la consommation de drogue, d'inactiver la drogue en présence d'anticorps spécifiques de la drogue et de produire de nouveaux anticorps spécifiques de la drogue, le vaccin éliminant ainsi l'effet de la drogue chez le patient et interrompant la relation de cause à effet entre stimulation de drogue et application de drogue, et l'effet d'élimination de l'activité de la drogue obtenu par la vaccination impliquant un effet s'induisant par une administration sous-cutanée de 40 microgrammes d'un conjugué morphine-BSA ou barbiturate-KLH répétée huit fois par intervalles de deux semaines.

2. Procédé suivant la revendication 1, **caractérisé en ce que** la drogue engendrant une dépendance relève de l'un des groupes suivants : opiacés, marihuana, amphétamines, cocaïne, barbituriques, sédatifs, méthaqualone, benzodiazépine, acide lysergique diéthylamide, drogues psychomimétiques, nicotine, drogues anticholinergiques et drogues antipsychotiques.

3. Procédé suivant la revendication 1, **caractérisé en ce que** la substance porteuse comporte un ou plusieurs des constituants chimiques suivants : protéines, glycoprotéines, polysaccharides, liposaccharides, lipoprotéines, métaux.

4. Procédé suivant la revendication 1, **caractérisé en ce que** la liaison entre la drogue engendrant une dépendance et l'haptène est par l'intermédiaire d'une substance formant pont.

5. Procédé suivant la revendication 1, **caractérisé en ce que** le vaccin est formulé avec addition d'un adjuvant de manière à renforcer l'effet immunogène du vaccin.

6. Procédé suivant la revendication 1, **caractérisé en ce que** l'on met le vaccin sous forme physiologiquement compatible par filtration stérile.

7. Procédé suivant la revendication 1, **caractérisé en ce que** l'on utilise une solution aqueuse dont le pH est fixé entre 5,5 et 8 par un tampon.

8. Procédé suivant la revendication 1, **caractérisé en ce que** le vaccin est soumis à dialyse une fois l'haptène lié à la substance porteuse, afin de le débarrasser des sous-produits de la fabrication.
